# EUROPEAN PATENT APPLICATION

(11) **EP 1 985 251 A2**
(43) Date of publication of application: **29.10.2008**
(21) Application number: 08008048.4
(22) Date of filing: 25.04.2008
(51) Int. Cl.: A61B 18/14, A61B 18/00, A61B 19/00

(54) **Endoscopic treatment tool**

(30) Priority: 27.04.2007 JP 2007119304
(71) Applicant: Olympus Medical Systems Corporation, Tokyo 151-0072 (JP)
(72) Inventor: Nakamura, Tsutomu, Tokyo (JP); Suzuki, Keita, Tokyo (JP)
(74) Representative: von Hellfeld, Axel

(57) **Abstract**

An endoscopic treatment tool (1) of the present invention includes a forceps (2) endoscopically inserted into a body cavity to perform a dissection treatment, a wire (3) in which the forceps (2) is connected to the distal end of the wire, and a sheath (4) in which the wire is passed through is made of an insulating material, a main body (9) in which the sheath (4) is fixed, and a slider (10) in which the wire (3) is fixed and is slidably disposed in an axial direction of the main body (9); a female screw (7) disposed on the sheath (4), which regulates the forward sliding movement of the wire (3) by abutting the stopper (6); a protruding length regulating member (8) exhibiting a insulating property that is provided with a through-hole in which the forceps (2) is passed through and is disposed on a distal end of the sheath (4) in a state in which at least a portion of the distal end member protrudes forward from the sheath (4); wherein the protruding length regulating member (8) permits a dissection portion covered length which is a distance between the distal end and a distal end of the sheath (4), to adjust with a plurality of different lengths. According to the present invention, a protrusion length of a dissection portion from the sheath can be adjusted with a plurality of different positions easily and in a reliable manner.

## Description

### BACKGROUND OF THE INVENTION

### Field of Invention

The present invention relates to an endoscopic treatment tool which is inserted into an instrument channel of an endoscopic apparatus.

### Description of the Related Art

Conventionally, a treatment tool is provided with, for example, such as a needle forceps which is endoscopically inserted into a body cavity and to excise mucosa etc, by transmitting a high-frequency current through the forceps. (See for example: Japanese Utility Model (Registration) Application, First Publication No. 61-191012 (Patent Document 1)). Such treatment tools are provided with a dissection portion to perform treatments, such as a needle forceps, and the dissection portion is disposed at a distal end of a wire passed through a sheath exhibiting insulating properties. The sheath is inserted into an instrument channel of an endoscope. The dissection portion is freely protruded and retracted from the distal end of the sheath by operating an operation member in which a proximal end of the wire is fixed.

In the treatment tool described above, the protruded length of the dissection portion is generally short and an adjustment of the protruded length has never been easy. In addition, since the endoscope is inserted into a body cavity with complex curving movements; hence operated amounts of the operation member and the protruded and retracted amounts of the distal end member do not often correspond. Therefore, the dissection portion can only be adjusted with the following two states: a state in which the dissection portion is completely protruded, and a state in which the dissection portion is housed in the sheath.

In order to overcome the conventional operational difficulties described above, an endoscopic treatment tool which permits a fine adjustment of the protruded length of the dissection portion by providing an anchor in which its diameter is larger than an inner diameter of the sheath on an electrode disposed in the sheath or an operation member so as to have resiliency upon advancing and retracting the dissection portion, is suggested. (See, for example: Japanese Patent Application, First Publication No. 2004-544 (Patent Document 2)).

However, when the protruded length of the dissection portion is adjusted in the dissecting tool disclosed in Patent Document 2, an operation is performed by watching a video image of a distal portion of an endoscope taken from a rear slant direction. In general, because the protruded length is adjusted with a small pitch such as 0.5mm, the problem still remains such that it is difficult to precisely adjust the protruded length to a desirable length.

### SUMMARY OF THE INVENTION

The present invention was conceived in view of the above-described circumstances and has as its objective the provision of an endoscipic treatment tool in which a protruded length of a dissection portion from a sheath can be adjusted with a plurality of states easily and precisely.

The endoscopic treatment tool according to a first aspect of the present invention includes a dissection portion endoscopically inserted into a body cavity to perform a dissection treatment, a wire in which the dissection portion is connected to the distal end of the wire, and a sheath in which the wire is passed through is made of an insulating material, a main body in which the proximal end of the sheath is fixed, a slider in which the proximal end of the wire is fixed and is slidably disposed in an axial direction of the main body; characterised in that a stopper that is disposed on the dissection portion or the wire and protrudes outward in a diameter direction of the wire; a forward movement regulating portion that is disposed on the sheath and abuts the stopper so as to regulate a forward sliding movement of the wire; a distal end member exhibiting an insulating property that is provided with a through-hole in which the dissection portion is passed through, and is disposed on a distal end of the sheath in a state in which at least a portion of the distal end member protrudes forward from the sheath; and the distal end member permits a dissection portion covered length which is a distance between the distal end and a distal end of the sheath, to adjust with a plurality of different lengths.

Note that in the present invention, the position and location in which a slider described hereinbelow is disposed is called 'a proximal side' or 'proximal end', and the position and location in which the dissection portion is disposed is called 'a distal side' or 'distal end'.

The endoscopic treatment tool of the present invention enables the dissection portion to protrude with a plurality of different lengths by adjusting the dissection portion covered length of the distal end member.

The endoscopic treatment tool of the present invention may be further provided with an engagement member which is inserted into the distal end of the sheath and a thread groove is provided on an inner surface of the engagement member, and the distal end member also includes an engaging portion provided with a thread which is engaged with the thread groove is disposed, so that the dissection portion covered length may be adjusted a plurality of different lengths by changing the engagement lengths of the engagement member and the distal end member.

An endoscopic treatment tool according to a second aspect of the present invention includes: a dissection portion endoscopically inserted into a body cavity to perform a dissection treatment; a wire in which the dissection portion is connected to the distal end of the wire; a sheath in which the wire is passed through is made of an insulating material; a main body in which a proximal end of the sheath is fixed; a slider in which the proximal end of the wire is fixed and is slidably disposed in an axial direction of the main body; and characterized in that a stopper that is disposed on the dissection portion or the wire and protrudes outward in the diameter direction; a forward movement regulating portion disposed on the sheath, which regulates the forward sliding movement of the wire by abutting the stopper; a plurality of distal end members exhibiting a insulating property provided with a through-hole in which the dissection portion is passed through, and is detachably fixed on the distal end of the sheath in a state in which at least a portion of the distal end members are protruded to the distal side of the sheath; and each of the plurality of distal end members has different lengths of a dissection portion covered length which is a distance between a distal end when the distal end members are fixed at the distal end of the sheath and the distal end of the sheath, so as to permit the dissection portion covered length adjusting with a plurality of different lengths by selecting any one of the distal end members and fixing it at the distal end of the sheath.

An endoscopic treatment tool according to a third aspect of the present invention is further including: a plurality of dissection portions with different lengths, which is endoscopically inserted into a body cavity to perform a dissection treatment; a plurality of wires in which each dissection portion is fixed at each distal end; a sheath in which the wire is passed through, is made of an insulating material; a main body in which the distal end of the sheath is fixed; and a plurality of sliders in which a proximal end of each wire is fixed, and are slidably disposed in an axial direction of the main body.

The endoscopic treatment tool of the present invention easily and reliably enables a protruded length of a dissection portion from the sheath to adjust with a plurality of different positions and held at these positions.

Accordingly, the present invention enables the protruded length of the dissection portion to adjust to a desirable length depending on, for example, the shape of a target tissue.

### BRIEF DESCRIPTION OF THE DRAWING

FIG. 1 shows a cross-section through a portion of the endoscopic treatment tool according to a first embodiment of the present invention.
FIG. 2 is an enlarged view of the endoscopic treatment tool in the vicinity of the distal portion.
FIG. 3 shows a view for describing a movement of the endoscopic treatment tool in the vicinity of the distal portion when in use.
FIG. 4 is a view for describing a state in which the endoscopic treatment tool is inserted into the endoscope.
FIG. 5A is an enlarged view of the endoscopic treatment tool in the vicinity of the distal portion thereof according to a second embodiment of the present invention.
FIG. 5B is an enlarged view for describing a state in which a different distal end member is disposed on the endoscopic treatment tool according to a second embodiment of the present invention.
FIG. 6 is an enlarged view of the endoscopic treatment tool in the vicinity of the distal portion thereof according to a third embodiment of The present invention.
FIG. 7 is a perspective view of an operating member of the endoscopic treatment tool of the present invention.

### DETAILED DESCRIPTION OF THE INVENTION

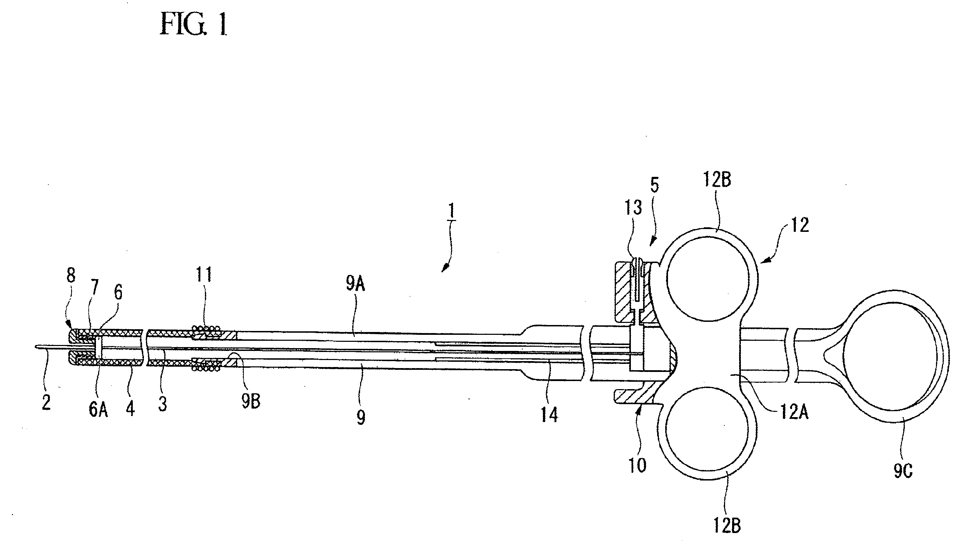

The first embodiment of the present invention of an endoscopic treatment tool (hereinafter abbreviated as simply 'a treatment tool') will be explained with reference to FIGS. 1 to 4.

FIG.1 shows a cross-section through a portion of an endoscopic treatment tool 1 of the present embodiment. The treatment tool 1 includes a wire 3 in which a high-frequency forceps (a dissection portion) 2 is disposed at a distal end thereof, a sheath 4 which covers an outer periphery of the wire 3, and an operation portion 5 to operate the wire 3 and the sheath 4.

The high-frequency forceps (hereinafter abbreviated as simply 'forceps') 2 is rod-shaped, with a length, for example 3 mm, of metallic member. The forceps performs, for example, an incision of tissues of body cavity, by transmitting a high-frequency current through the forceps. The shape of forceps 2 may not be limited to a rod shape; other shapes such as a spatula shape and a hook shape may also be employed.

The wire 3 is formed of a metal such as stainless steal, and inserted into the sheath 4 which is described later.

A stopper 6 provided with a protruded portion 6A projecting outward in the radial direction with respect to the forceps 2 is interposed between a distal end of the wire 3 and a proximal end of the forceps 2. The position of the stopper 6 is not limited therebetween, and the stopper 6 may also be disposed on the wire 3 or disposed on the forceps 2. Alternatively, a part of the stopper 6 may also protrude outward in the radial direction as the protruded portion 6A, or the stopper 6 may also be formed in a cylindrical shape in which the diameter is larger than the diameter of the forceps 2 so as to form the protruded portion 6A in the entire circumference.

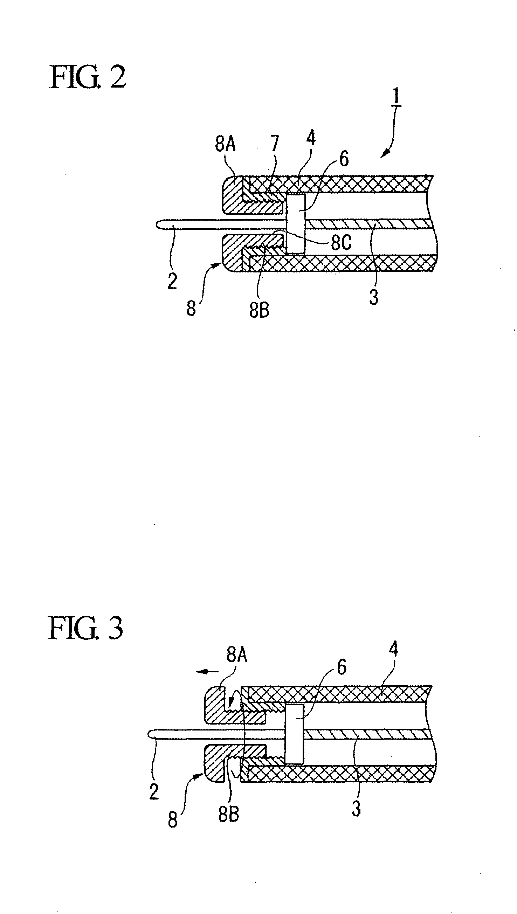

The sheath 4 is a tubular member formed of, for example, a resin exhibiting both an insulating property and flexibility. As shown in FIG. 2 of the enlarged diagram, a female screw (a forward movement regulating portion, an engagement member) 7 is fixed by press-fitting on a distal end of the sheath 4, and a protruding length regulating member (a distal end member) 8 which regulates a protruded length of the forceps 2 at a desirable length is engaged with the female screw 7.

The female screw 7 is substantially a cylindrical-shaped member formed of a metal, a resin and so on, and is provided with a thread groove on an inner surface thereof. The protruding length regulating member 8 is formed of a resin, rubber or the like exhibiting an insulating property, and includes a disk-shaped portion 8A of a substantially disk shape disposed on the outside of the sheath 4, and a pipe-like portion (engagement portion) 8B engaged with the female screw 7. Furthermore, a through-hole 8C in which the forceps 2 is passed therethough is formed in the center of the protruding length regulating member 8.

A thread which engages with a thread groove of the female screw 7 is formed on the outer periphery surface of the pipe-like portion 8B.

The pipe-like portion 8B is colored with different colors; for example with three different colors i.e., red, yellow and blue; each with a predetermined length, for example 0.5 mm, from the disk portion 8A side.

In addition, according to the treatment tool 1 of the present embodiment, the entire pipe-like portion 8B mounts the protruding length regulating member 8 so as to threadably engage with the female screw 7, the forceps 2 is set to protrude from the distal end of the protruding length regulating member 8 by, for example, 2.0 mm, when the wire 3 is advanced until the stopper 6 comes into a contact with the female screw 7.

As shown in FIG. 1, an operator portion 5 includes a main body 9 in which the sheath 4 is fixed and a slider 10 in which the wire 3 is fixed.

The main body 9 is a rod-shaped member provided with a guide chase 9A therein facilitating the slider 10 to slide in an axial direction. A through-hole 9B is provided at the distal end of the main body 9 facilitating the wire 3 to pass therethrough, and the proximal end of the sheath 4 is fixed by fixing aids, such as a coil 11. A ring 9C for fingers to be suspended when carrying out a procedure is disposed at a proximal end of the main body 9.

A slider 10 includes a plug 13 which is connected to a high-frequency power source not illustrated, connected to an operator member 12 provided with a cylindrical member 12A which surrounds the outer periphery of the main body 9 and a handle 12B in which the finger is suspended when carrying out a procedure. A proximal end of the wire 3 is penetrated through a buckling prevention pipe14 formed of a rigid material. Both proximal ends of the buckling prevention pipe 14 and the wire 3 are connected and fixed to the plug 13 inside of the guide chase 9A by fixing aids such as a thread not illustrated. Thus, the slider 10 and the wire 3 are slidably disposed on the main body 9 in an axial direction along the guide chase 9A.

An operational movement of the treatment tool 1 configured as above is described hereinbelow.

First, an operator rotates the protruding length regulating member 8 so as to adjust the protruding length of the forceps 2 at the time of operation. In particular, the pipe-like portion 8B is made to protrude from the distal end of the sheath 4 by a desirable length by rotating the disk-shaped portion 8A as shown in FIG. 3.

The above operation facilitates the change of the engagement length of the pipe-like portion 8B and the female screw 7 so as to change a dissection portion covered length which is a length of the proximal end side of the forceps 2 covered by the protruding length regulating member 8. At this time, if the adjustment is carried out with the color of the pipe-like portion 8B protruded from the sheath 4 as a guide, the protruded length of the forceps 2 can be controlled approximately at every 0.5mm.

After the adjustment of the protruding length regulating member 8 is completed, an insertion portion of an endoscope is inserted into a body cavity of, for example, a patient, then a distal end of the insertion portion is moved to in the vicinity of a treatment target tissue.

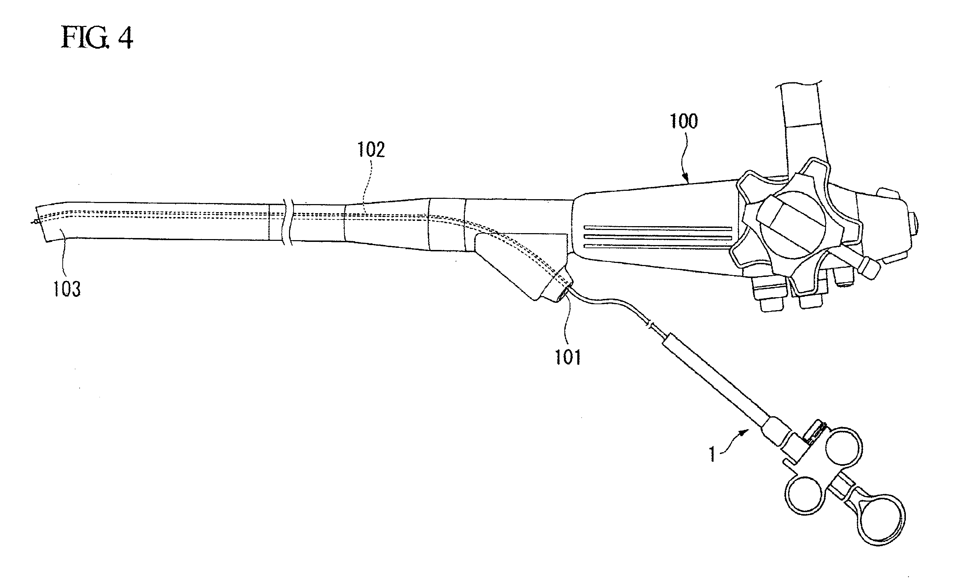

Next, the slider 10 of the treatment tool 1 is completely pulled toward the proximal side (the ring 9C side) so as to retract, and the forceps 2 is accommodated in the sheath 4. As shown in FIG. 4, the distal end of the sheath 4 is inserted into an instrument channel 102 via a forceps port 101 which opens on an operator portion of an endoscope 100, and the distal end of the treatment tool 1 is made to protrude from a distal end of an insertion portion 103. A cable, not illustrated, is then connected to the plug 13. The cable may also be connected in advance prior to the treatment tool 1 being inserted into the endoscope 100.

At this state, the operator presses the slider 10 so as to advance the wire 3 till the stopper 6 comes into contact with the proximal end of the female screw 7. Then, the stopper 6 abuts the female screw 7 so that the forward sliding movement of the wire 3 is regulated, resulting in the forceps 2 being protruding from the protruding length regulating member 8 at a desirable length. Then, a high-frequency current is supplied to the forceps 2, and a treatment of a target tissue, for example, incision and excision is performed.

If the protruded length of the forceps 2 is changed during the treatment, the treatment tool 1 is first pulled out from the endoscope 100, then the protruding length regulating member 8 is adjusted again.

According to the treatment tool 1 of the present embodiment, the dissection portion covered length of the forceps 2 can be adjusted at a plurality of different lengths easily and reliably by rotating the protruding length regulating member 8 so as to change the relative position with respect to the sheath 4. By virtue of this feature, a treatment can be performed by adjusting the protruded length of the forceps 2 depending on, for example, a shape of a target tissue.

Furthermore, in the preceding embodiments, the pipe-like portion 8B is colored, however it is also acceptable to mark, for example, a scale onto the forceps 2, instead. In this way, the protruding length regulating member 8 can be adjusted with the scale on the forceps 2 as a guide, in a state in which the stopper 6 is abutted to the female screw 7.

Furthermore, in the preceding embodiments, the engaged member is a female screw; however it is also acceptable to provide a thread portion in which the engagement member protrudes from the distal end of the sheath, and a concave portion provided with a thread groove which engages with the thread portion disposed at the proximal end side of the protruding length regulating member.

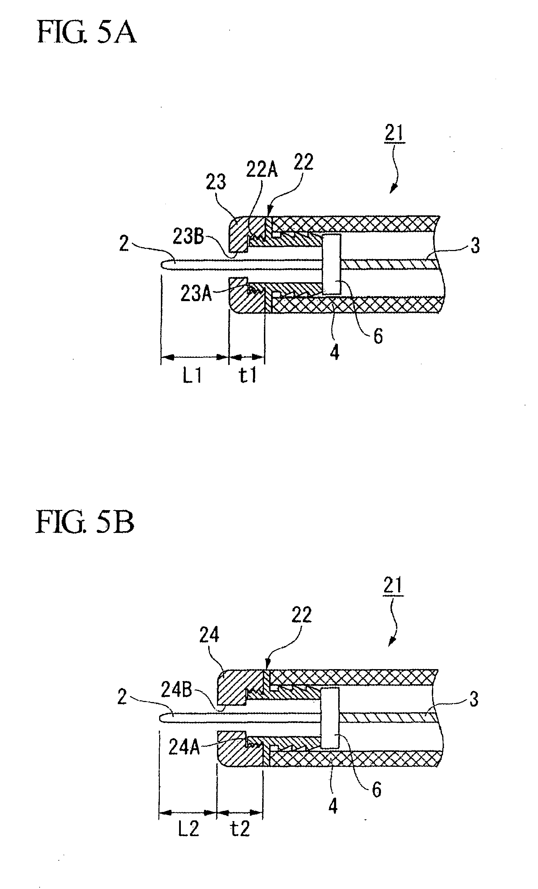

Next, a second embodiment of the present invention of the treatment tool will be explained with reference to FIGS. 5A and 5B. The difference between the endoscopic treatment tool 1 of the first embodiment described above and a treatment tool 21 of the present embodiment is that the treatment tool 21 is provided with a plurality of replaceable distal end members. In the following description, components that are the same as the first embodiment shall be provided with the same numeric symbols and redundant descriptions shall be omitted.

FIG. 5A is an enlarged diagram in the vicinity of a distal end of the treatment tool 21. A fitting member 22 provided with a pipe shaped thread 22A in which a thread ridge is disposed on an outer periphery thereof is fixed by, for example, press-fitting, is disposed at the distal end of the sheath 4. The forceps 2 can be protruded from the distal end of the thread 22A by passing through the fitting member 22.

The treatment tool 21 includes two types of distal end members 23 and 24 which are formed of the same material of the protruding length regulating member 8 and both members exhibit insulating properties. The distal end members 23 and 24 can be detachably attached and fitted to the fitting member 22 disposed at the distal end of the sheath.

FIG. 5A shows a state in which the distal end member 23 is fitted to the fitting member 22. A concave portion 23A provided with a thread groove which is engaged with the thread ridge of the thread 22A is formed on a proximal side surface of the distal end member 23 (the side which is fitted with the fitting member 22). A though-hole 23B is provided along a central axial line, and the forceps 2 can be protruded from the though-hole 23B.

A protrusion length L1 of the forceps 2 from the distal end member 23 is set as 2.0 mm when the distal end member 23 is fitted to the fitting member 22.

FIG. 5B shows a state in which another distal end member 24 is fitted on the fitting member 22. The shape of the distal end member 24 is substantially the same as the distal end member 23, provided with a concave portion 24A and a though-hole 24B which are substantially the same shapes of the concave portion 23A and the though-hole 23B, respectively.

Note that a dissection portion covered length t2 which is a length between a distal end of the distal end member 24 and the distal end of the sheath 4 is set longer than a dissection portion covered lengtht1 of the distal end member 23 by 0.5 mm. Thus, a protruded length L2 of the forceps 2 when the distal end member 24 is attached is set as 1.5 mm which is shorter than the protruded length L1 described above by 0.5mm.

In addition, each of the distal end members 23 and 24 may be colored with different colors for ease of identification.

An operational movement of the treatment tool 21 configured as above is described hereinbelow.

First, an endoscope is inserted into a body cavity of a patient in the same manner as described in the first embodiment. Prior to inserting the treatment tool 21 into the forceps port 101, either of the distal end members 23 and 24 is fitted onto the fitting member 22 in order to set a protruding length of the forceps 2 to a desirable length. After the distal end member is fitted, a distal end of the treatment tool 21 is inserted via the forceps port 101 so as to protrude from a distal end of the insertion portion 103.

When an operator pushes the slider 10 forward to make the stopper 6 abut the proximal end of the fitting member 22, a protruded length of the forceps 2 becomes a desirable length.

When the protruded length of the forceps 2 is adjusted, the operator pulls the treatment tool 21 out from the endoscope 100 in the same manner described in the first embodiment; the distal end member is replaced with another distal end member and fit onto the fitting member 22. The operation is continued by inserting the treatment tool 21 into the endoscope 100 again.

According to the treatment tool 21 of the present embodiment, the protruded length of the forceps 2 can be adjusted easily by changing the dissection portion covered length by only replacing a plurality of the distal end members and fitting it onto the fitting member. Therefore the adjustment of the protruded length of the forceps 2 can be performed easily without a fine skilled operation.

In the present embodiment, the embodiment with the two types of replaceable distal end members is described; however the present embodiment is not limited thereto. It is also acceptable to provide more than three types of the distal end members depending on different protrusion lengths of the forceps 2 required for these adjustments. Furthermore, the dissection portion covered length of each distal end member may also be set at a desirable length.

Alternatively, the distal end member and the fitting member are fitted with the corresponding thread ridge and thread groove in the present embodiment; it is also acceptable if the distal end members are fixed by other conventionally known fitting mechanisms or fitting aids, instead.

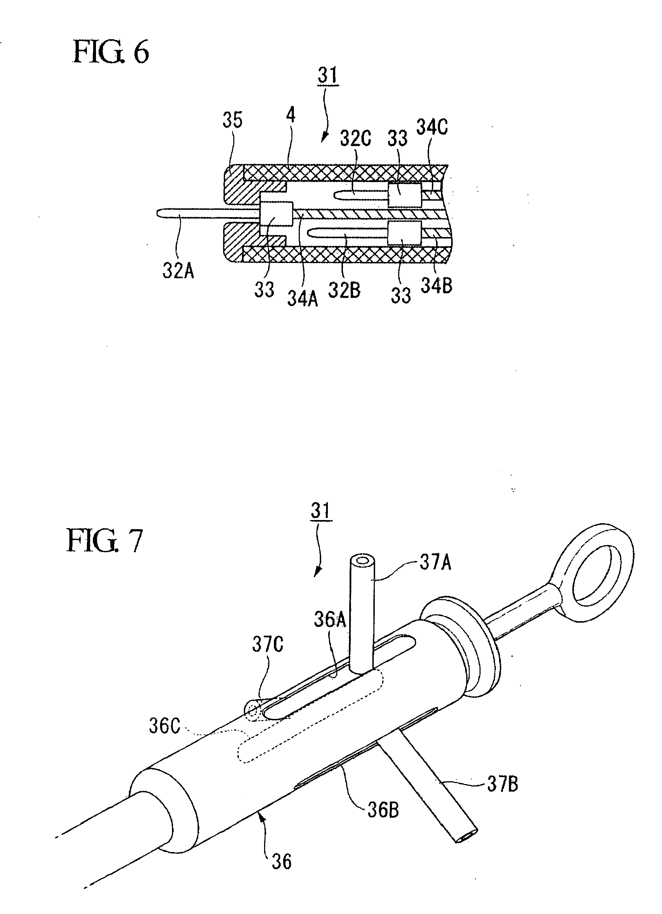

Next, a treatment tool according to a third embodiment of the present invention will be explained with reference to FIGS. 6 and 7. The difference between a treatment tool 31 of the present embodiment and the endoscopic treatment tool 1 described above is a plurality of wires and forceps are included.

In the following description, components that are the same as the first embodiment shall be provided with the same numeric symbols and redundant descriptions shall be omitted.

FIG. 6 is an enlarged diagram in the vicinity of a distal end of a treatment tool 31. The treatment tool 31 includes three forceps with different lengths 32A, 32B and 32C, and each forceps is connected to each wire 34A, 34B and 34C interposing same-shaped stoppers 33 between the proximal end of each of the forceps and the wire.

An abutting member (forward movement regulating portion) 35 which abuts the stopper 33 so as to regulate sliding of each wire to the forward direction is fixed by, for example, press-fitting at the distal end of the sheath 4.

Lengths of each forceps 32A, 32B and 32C are set so as to protrude from the distal end of the abutting member 35 by, for example, 2.0mm, 1.5mm and 1.0mm, respectively, when the stopper 33 comes into contact to the abutting member 35.

FIG. 7 is a perspective view of the treatment tool 31. A main body 36 is a rod-shaped member provided with three guide grooves 36A, 36B and 36C on a lateral face of the main body 36 at equal distance apart, extending along the longitudinal direction. Pipe shaped sliders 37A, 37B and 37C provided with a flange, not illustrated, are slidably disposed on each groove.

Plugs 38A, 38B and 38C (not illustrated) which are connected to a high-frequency power source, not illustrated, are fixed on each slider. Proximal ends of the wires 34A, 34B and 34C are fixed onto each plug in the same manner described above in the first embodiment. It is preferable to mark a protruded length of a connected forceps on a surface of each slider by, for example, carving if necessary so as to aid the identification of the forceps. In addition to the marking of the protruded length, it is also acceptable to color each slider with a different color so as to improve the identification of the forceps.

An operational movement of the treatment tool 31 configured as above is described hereinbelow.

First, the distal end of the treatment tool 31 is made to protrude from a distal end of the insertion section 103 of the endoscope 100 in the same manner as described in the first embodiment.

An operator selects one of the forceps according to a desirable protrusion length and a corresponding plug is connected to a high-frequency power source. Upon sliding the slider in which the plug is fixed to the forward direction, the connected wire and forceps advance, and the stopper 33 comes into contact with the abutting member 35 so as to stop. At this time, a state in which the forceps protrudes from the distal end of the abutting member 35 by the desirable length is achieved, so that the operator can perform various treatments.

When changing the protruded length of the forceps, the advanced slider is retracted to house the forceps into the sheath 4. Then the plug is replaced by a different plug which is corresponded to a new target forceps, and is connected to the high-frequency power source. Similarly, a slider in which the new plus is fixed is sided forward as described above so as to make the slider protrude from the abutting member 35.

According to the treatment tool 31 of the present embodiment, the three types of forceps with different lengths are fixed to each slider via the wire, a protrusion length of the forceps can be adjusted with a plurality of different positions by simply replacing the slider. Therefore, there is no need to withdraw the treatment tool from the endoscope every time changing of the protrusion length of a forceps is required; the treatment tool of the present invention allows a change of the protrusion length of a forceps while the distal end of the sheath 4 remains in a body cavity of a patient and so on.

While preferred embodiments of the invention have been described and illustrated above, it should be understood that these are exemplary of the invention and are not to be considered as limiting. Additions, omissions, substitutions, and other modifications can be made without departing from the scope of the invention. Accordingly, the invention is not to be considered as being limited by the foregoing description, and is only limited by the scope of the appended claims.

For example, the stopper and the forward movement regulating member abut at the in the vicinity of the distal end of the sheath is explained in the above embodiment, however the present invention is not limited thereto; for example, it is also acceptable if the forward sliding of the wire is regulated by abutting the stopper and the forward movement regulating member further to the rear position of the sheath.

Note that the middle section of an outer sheath is often curved since the treatment tool is used by insertion into an endoscope. Therefore, it is preferable to abut the stopper and the forward movement regulating member in the vicinity of the distal end of the sheath where it is not easily bent, in order to adjust the protrusion length of the forceps in a reliable manner.

## Claims

1. An endoscopic treatment tool 1 comprising:
a dissection portion 2 endoscopically inserted into a body cavity to perform a dissection treatment;
a wire 3 in which the dissection portion is connected to a distal end of the wire;
a sheath 4 in which the wire is passed through is made of an insulating material;
a main body 9 in which the proximal end of the sheath is fixed; and
a slider 10 in which the proximal end of the wire is fixed and is slidably disposed in an axial direction of the main body;
wherein a stopper 6 is disposed on the dissection portion or the wire and protrudes outward in a diameter direction of the wire;
a forward movement regulating portion 7 is disposed on the sheath and abuts the stopper so as to regulate a forward sliding movement of the wire;
a distal end member 8 exhibiting a insulating property is provided with a through-hole in which the dissection portion is passed through, and is disposed on a distal end of the sheath in a state in which at least a portion of the distal end member protrudes forward from the sheath; and
the distal end member permits a dissection portion covered length which is a distance between the distal end and the distal end of the sheath, to adjust with a plurality of different lengths.

2. The endoscopic treatment tool according to Claim 1, further comprising:
an engagement member 7 which is inserted into the distal end of the sheath and a thread groove is provided on an inner surface of the engagement member;
the distal end member provided with an engaging portion 8B provided with a thread which is engaged with the thread groove; and
the dissection portion covered length is adjusted to a plurality of different lengths by changing the engagement lengths of the engagement member and the distal end member.

3. An endoscopic treatment tool 21 comprising:
a dissection portion 2 endoscopically inserted into a body cavity to perform a dissection treatment;
a wire 3 in which the dissection portion is connected to the distal end of the wire;
a sheath 4 in which the wire is passed through is made of an insulating material;
a main body 9 in which a proximal end of the sheath is fixed; and
a slider 10 in which the proximal end of the wire is fixed and is slidably disposed in an axial direction of the main body;
wherein a stopper 6 that is disposed on the dissection portion or the wire and protrudes outward in a radial direction,
a forward movement regulating portion 7 that is disposed on the sheath which regulates the forward sliding movement of the wire by abutting the stopper;
a plurality of distal end members 23, 24 exhibiting a insulating property provided with a through-hole in which the dissection portion is passed through, and is detachably fixed on the distal end of the sheath in a state in which at least a portion of the distal end members are protruded from the sheath; and
each of the plurality of distal end members has a different length of a dissection portion covered length which is a distance between a distal end when the distal end members are fixed at the distal end of the sheath and the distal end of the sheath, so as to permit the dissection portion covered length adjusting with a plurality of different lengths by selecting any one of the distal end members and fixing the distal end member at the distal end of the sheath.

4. An endoscopic treatment tool 21 comprising:
a plurality of dissection portions 32A, 32B and 32C with different lengths, which is endoscopically inserted into a body cavity to perform a dissection treatment,
a plurality of wires 34A, 34B and 34C in which each of the dissection portions is fixed at each of the distal ends,
a sheath 4 in which the wire is passed through, the sheath 4 being made of an insulating material,
a main body 36 in which the distal end of the sheath is fixed, and
a plurality of sliders 37A, 37B and 37C in which a proximal end of each wire is fixed and are slidably disposed in an axial direction of the main body.
